# FASCICULE DE BREVET EUROPEEN

(11) **EP 3 142 521 B1**
(45) Date de publication et mention de la délivrance du brevet: **17.10.2018**
(21) Numéro de dépôt: 15728052.0
(22) Date de dépôt: 13.05.2015
(51) Int. Cl.: A47G 25/90

(54) **SYSTEME D'ASSISTANCE POUR ENFILER UN DISPOSITIF DE COMPRESSION MEDICALE SUR UN MEMBRE D'UN PATIENT**
HILFSSYSTEM ZUM ANBRINGEN EINER MEDIZINISCHEN KOMPRESSIONSVORRICHTUNG AUF EINEM GLIED EINES PATIENTEN
ASSISTANCE SYSTEM FOR FITTING A MEDICAL COMPRESSION DEVICE ONTO A LIMB OF A PATIENT

(30) Priorité: 16.05.2014 FR 1454414
(43) Date de publication de la demande: 22.03.2017
(73) Titulaire: Belovia, 71400 Autun (FR)
(72) Inventeur: GOGUE-MEUNIER, Guillaume, 71540 Reclesne (FR); TRUONG, Romain, 71400 Autun (FR); M. CHEVALIER, Joël, 21430 Sussey (FR)
(74) Mandataire: Cabinet Plasseraud
(86) Numéro de dépôt international: PCT/FR2015/051249
(87) Numéro de publication internationale: WO 2015/173512

(56) Documents cités:
- WO-A1-2012/032459
- FR-A1- 2 921 552
- US-A1- 2007 084 890

## Description

L'invention se rapporte à un système d'assistance pour enfiler un dispositif de compression médicale sur un membre d'un patient.

L'invention s'applique notamment à l'enfilage d'un dispositif de compression médicale se présentant sous la forme d'un bas de compression médicale ou bas de contention sur un membre inférieur du patient.

Les bas de contention sont utilisés chez des patients, généralement âgés, notamment dans le cadre du traitement de pathologies vasculaires et, en particulier, dans le cadre du traitement de la maladie veineuse chronique des membres inférieurs.

L'enfilage d'un bas de contention nécessite de fournir un effort important pour écarter suffisamment le bas de contention de manière à permettre au pied de passer, puis pour faire glisser le bas de contention le long de la jambe. Cet effort peut être d'autant plus important que le membre sur lequel le bas de contention doit être placé est humide, notamment après la toilette du patient, ou recouverte d'un produit, d'une couche textile ou autre, notamment après des soins pratiqués sur le patient. Outre son importance, l'effort peut avoir à être exercé selon différentes orientations parfois peu naturelles, notamment lorsque le patient a perdu de la souplesse et ne peut tendre le pied et/ou lever la jambe.

Les patients eux-mêmes ne disposant généralement plus de la dextérité, de la force et de la souplesse nécessaires pour l'enfilage du bas de contention, ce geste peut être confié à un aidant tel qu'une aide-soignante, une aide à domicile, une infirmière ou autre.

Toutefois, l'aidant peut être amené à répéter un grand nombre de fois ce geste pénible physiquement. Une telle répétition peut conduire à des troubles musculaires et/ou articulaires chez l'aidant et peut dissuader l'aidant de pratiquer l'ensemble du traitement et des soins nécessaires au patient.

Pour simplifier l'enfilage du bas de contention tant par le patient que par l'aidant, des systèmes d'assistance, connus sous le nom d'enfile-bas, ont été conçus.

De tels systèmes d'assistance sont, par exemple, décrits dans les documents FR 2 818 883 et FR 2 951 059. Ils comprennent deux organes d'étirage montés sur un support en regard l'un de l'autre et déplaçables l'un par rapport à l'autre en translation. Chaque organe d'étirage comporte une ou plusieurs parties de retenue sur chacune desquelles au moins une partie du bas de contention, au voisinage d'une ouverture d'enfilage de celui-ci, peut être positionnée lorsque les organes d'étirage sont rapprochés l'un de l'autre, et étirée lorsque les organes d'étirage sont écartés l'un de l'autre.

Toutefois, les systèmes d'assistance connus demandent toujours à l'utilisateur, patient ou aidant, d'exercer un effort important pour écarter suffisamment les organes d'étirage et les maintenir en position de manière à permettre l'enfilage du bas de contention.

De la même manière, le document US 2007/0084890 décrit un système d'assistance selon le préambule de la revendication 1 comprenant des organes d'étirage dont la conformation nécessite de les écarter l'un de l'autre de façon importante pour permettre le passage du membre et, en particulier, du pied.

L'invention vise à améliorer les systèmes d'assistance connus.

A cet effet, l'invention propose un système d'assistance pour enfiler un dispositif de compression médicale sur un membre d'un patient, le dispositif de compression médicale présentant une ouverture d'enfilage, le système d'assistance comprenant :
- un support s'étendant selon un axe longitudinal,
- deux organes d'étirage comprenant chacun un bras de liaison s'étendant depuis le support selon une première direction transversale, et un corps s'étendant depuis le bras de liaison selon une deuxième direction transversale, le corps de chacun des organes d'étirage comportant une base reliée au bras de liaison et au moins une partie de retenue écartée de la base et sur laquelle au moins une partie du dispositif de compression médicale au voisinage de l'ouverture d'enfilage est destinée à être positionnée, les organes d'étirage étant montés sur le support en regard l'un de l'autre et déplaçables l'un par rapport à l'autre en translation selon l'axe longitudinal,
dans lequel la partie de retenue et la base d'au moins l'un, premier, des organes d'étirage s'étendent respectivement dans des plans transversaux perpendiculaires à l'axe longitudinal, le plan transversal contenant la partie de retenue du premier organe d'étirage étant décalé selon l'axe longitudinal en direction de la partie de retenue de l'autre, deuxième, organe d'étirage par rapport au plan transversal contenant la base dudit premier organe d'étirage, le corps du premier organe d'étirage présentant un évidement selon la deuxième direction transversale au moins au niveau de la partie de retenue,
et dans lequel le premier organe d'étirage comprend :
- une tige mise en forme pour former le corps et comprenant la base du corps,
- un élément de liaison extérieur s'étendant selon la première direction transversale pour former une partie du bras de liaison, la base du corps étant reliée à l'élément de liaison extérieur, et
- un élément de liaison intérieur s'étendant selon la première direction transversale parallèlement à l'élément de liaison extérieur, l'élément de liaison intérieur étant écarté de l'élément de liaison extérieur selon l'axe longitudinal en direction du deuxième organe d'étirage, l'élément de liaison intérieur formant une autre partie du bras de liaison à laquelle la tige est reliée à l'opposé de la base.

Ainsi, les organes d'étirage peuvent être placés en une pluralité de positions relatives dans chacune desquelles les bases des organes d'étirage sont écartées l'une de l'autre d'un écartement supérieur à un écartement entre les parties de retenue. Un tel élargissement du passage entre les bases des organes d'étirage combiné à l'évidement d'au moins l'un des organes d'étirage permet d'assurer l'introduction du membre, et notamment du pied, du patient entre les organes d'étirage tout en réduisant l'écartement nécessaire entre les organes d'étirage pour enfiler le dispositif de compression médicale. Les efforts nécessaires pour écarter et maintenir le dispositif de compression médicale peuvent ainsi être réduits.

Le corps du premier organe d'étirage peut présenter une concavité orientée dans une direction opposée au deuxième organe d'étirage au niveau de la partie de retenue dudit premier organe d'étirage.

En particulier, la partie de retenue du premier organe d'étirage peut présenter un pli s'étendant selon la première direction transversale.

Le corps de chacun des organes d'étirage peut présenter une extrémité distale opposée à la base, la partie de retenue du premier organe d'étirage étant décalée selon l'axe longitudinal en direction de la partie de retenue du deuxième organe d'étirage par rapport à l'extrémité distale dudit premier organe d'étirage.

L'extrémité distale du premier organe d'étirage peut être disposée entre deux plans transversaux dans lesquels s'étendent respectivement la base et la partie de retenue dudit premier organe d'étirage.

Dans un mode de réalisation, le premier organe d'étirage peut être formé d'un seul tenant par la tige mise en forme (notamment pliée ou cintrée), la tige comportant une branche extérieure formant l'élément de liaison extérieur, et un arceau s'étendant globalement selon la deuxième direction transversale pour former le corps, l'arceau comprenant une branche de base s'étendant dans le prolongement de la branche extérieure pour former la base du corps.

La tige peut alors comporter en outre une branche intérieure formant l'élément de liaison intérieur.

La tige peut être réalisée en acier ressort.

La tige peut présenter une section ronde.

Ces dispositions permettent aux organes d'étirage d'être déformés élastiquement pour disposer d'une certaine souplesse. Dans chacune des positions relatives des organes d'étirage, les corps peuvent ainsi être écartés l'un de l'autre avec un certain jeu.

La partie de retenue de chacun des organes d'étirage peut comporter deux bords de retenue en regard écartés l'un de l'autre selon la première direction transversale, chacun des bords de retenue présentant une concavité orientée dans une direction opposée à l'autre bord de retenue.

Le corps de chacun des organes d'étirage peut comporter une pluralité de parties de retenue présentant chacune un écartement entre les bords de retenue, l'écartement entre les bords de retenue de l'une des parties de retenue située à proximité de la base étant supérieur à l'écartement entre les bords de retenue de l'une des parties de retenue située à distance de la base.

Selon des dispositions particulières, la réduction des efforts nécessaires pour étirer le dispositif de compression médicale permet d'automatiser le système d'assistance tout en le gardant portable à la main par l'utilisateur.

Le système d'assistance peut comprendre en outre un dispositif d'entraînement logé dans le support et adapté pour déplacer les organes d'étirage l'un par rapport à l'autre, et un organe d'activation placé sur le support et actionnable par un utilisateur pour activer le dispositif d'entraînement.

Le support peut être adapté pour être porté à la main par l'utilisateur.

D'autres objets et avantages de l'invention apparaîtront à la lecture de la description qui suit d'un mode de réalisation particulier de l'invention donné à titre d'exemple non limitatif, la description étant faite en référence aux dessins annexés dans lesquels :
- la figure 1 est une représentation en perspective selon une première orientation d'un système d'assistance pour enfiler un dispositif de compression médicale sur un membre d'un patient selon un mode de réalisation de l'invention, le système d'assistance comprenant un corps sur lequel deux organes d'étirage sont montés déplaçables en translation l'un par rapport à l'autre, les organes d'étirage étant dans une première position relative, rapprochée,
- la figure 2 est une représentation en perspective selon une deuxième orientation du système d'assistance de la figure 1, les organes d'étirage étant dans une deuxième position relative, écartée, et un dispositif d'entraînement des organes d'étirage étant représenté en transparence du corps,
- les figures 3 à 5 sont des représentations en plan respectivement selon trois orientations différentes de l'un des organes d'étirage du système d'assistance de la figure 1,
- les figures 6 et 7 sont des représentations en plan respectivement selon deux orientations différentes des organes d'étirage du système d'assistance de la figure 1 dans les première (en traits pleins) et deuxième (en traits mixtes) positions relatives,
- la figure 8 est une représentation en perspective illustrant l'enfilage d'un dispositif de compression médical sur un membre d'un patient à l'aide du système d'assistance de la figure 1.

Sur les figures, les mêmes références désignent des éléments identiques ou analogues.

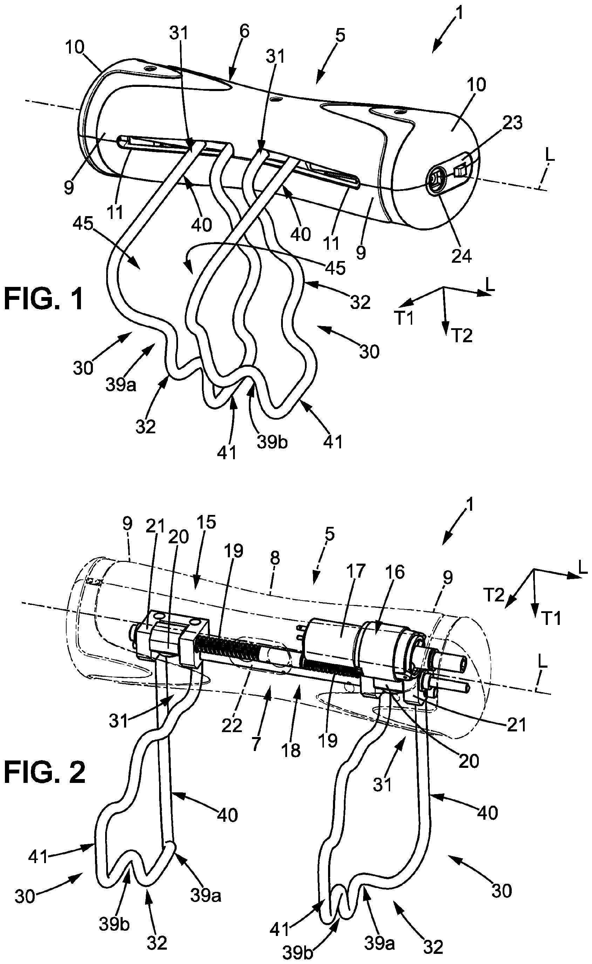

Les figures 1 et 2 représentent un système d'assistance 1 permettant de simplifier l'enfilage d'un dispositif de compression médicale sur un membre d'un patient. Dans le mode de réalisation représenté, sans y être limité, le système d'assistance 1 est utilisé pour l'enfilage d'un bas de compression médicale ou bas de contention 4 sur un membre inférieur 3 du patient.

Sur la figure 1, le système d'assistance 1 comprend un support 5 s'étendant selon un axe longitudinal L et adapté pour être porté à la main par un utilisateur, patient ou aidant. Le support 5 comporte une paroi latérale 6, tubulaire autour de l'axe longitudinal L, définissant un logement intérieur 7. Dans le mode de réalisation représenté, la paroi latérale 6 présente une partie centrale 8 amincie et s'évase en s'écartant de la partie centrale 8 vers deux extrémités 9 opposées. La paroi latérale 6 présente alors généralement une forme en hyperboloïde permettant d'améliorer la prise en main par l'utilisateur. Les extrémités 9 de la paroi latérale 6 peuvent être fermées par exemple au moyen de parois transversales 10. Le support 5 peut alors être constitué de deux demi-coques assemblées l'une à l'autre de manière amovible le long d'un plan de joint longitudinal, c'est-à-dire comprenant l'axe longitudinal L, afin de permettre d'accéder au logement intérieur 7. En variante, l'accès au logement intérieur 7 du support 5 pourrait être obtenu de toute autre manière appropriée, par exemple au moyen d'au moins un capot amovible portant l'une des parois transversales 10. Le support 5 présente également deux fentes 11 ménagées sur la paroi latérale 6 pour faire communiquer le logement intérieur 7 avec l'extérieur. Chaque fente 11 s'étend selon l'axe longitudinal L sur une partie d'une longueur, mesurée selon l'axe longitudinal L, d'une moitié du support 5. En variante, une seule fente s'étendant sur une partie de la longueur du support 5 pourrait être prévue.

Comme représenté sur la figure 2 en transparence du support 5, un dispositif d'entraînement 15 est disposé dans le logement intérieur 7. Le dispositif d'entraînement 15 comprend au moins un actionneur 16, se présentant dans le mode de réalisation représenté sous la forme d'un moteur électrique 17 relié à une alimentation électrique de préférence portable telle qu'une batterie (non représentée), et un système de transmission 18, se présentant dans le mode de réalisation représenté sous la forme d'un système vis-écrou. Le système de transmission 18 comprend alors deux portions filetées 19 s'étendant parallèlement à l'axe longitudinal L et reliées au moteur électrique 17 pour pouvoir être entraînées en rotation. Le système de transmission 18 comprend également deux écrous 20 bloqués en rotation par rapport au support 5 et montés respectivement sur les portions filetées 19. Chacun des écrous 20 est monté sur un chariot 21 disposé en regard de l'une des fentes 11.

De cette manière, une rotation de chacune des portions filetées 19 entraîne un déplacement en translation selon l'axe longitudinal L de l'écrou 20 et du chariot 21 associé montés sur cette portion filetée 19. Dans le mode de réalisation représenté, les deux portions filetées sont prévues sur la même tige filetée et comportent respectivement des filetages à pas opposés. Chacune des portions filetées 19 est ainsi entraînée conjointement avec l'autre portion filetée 19 pour permettre un déplacement conjoint des chariots 21 dans des sens opposés de rapprochement ou d'écartement.

Un organe d'activation 22 sous la forme de deux boutons de commande, un pour chaque sens de déplacement des chariots 21, peut alors être placé sur le support 5. Chacun des boutons de commande 22 est actionnable par l'utilisateur pour activer le dispositif d'entraînement 15 et déplacer les chariots 21 selon l'axe longitudinal L, dans un sens ou dans l'autre.

En variante, chacune des portions filetées 19 pourrait être entraînée indépendamment de l'autre portion filetée 19 pour permettre un déplacement de l'un des chariots 21 indépendamment de l'autre chariot 21. En outre, l'organe d'activation 22 pourrait présenter toute autre forme appropriée et comprendre, par exemple, un seul ou plus de deux boutons de commande dont l'actionnement entraîne le déplacement souhaité des chariots 21.

Comme représenté sur la figure 1, afin de pouvoir arrêter et mettre sous tension le dispositif d'entraînement 15, un interrupteur 23 actionnable par l'utilisateur peut être prévu sur le support 5, par exemple sur l'une des parois transversales 10. Un connecteur 24 relié à la batterie du dispositif d'entraînement 15 peut également être prévu sur le support 5, par exemple sur l'une des parois transversales 10, pour pouvoir recharger la batterie.

L'invention décrite avec un dispositif d'entraînement 15 électrique comprenant un moteur électrique 17, une batterie et un système de transmission 18 vis-écrou pourrait être mise en oeuvre avec tout autre dispositif d'entraînement, électrique, pneumatique, hydraulique ou autre, comprenant tout actionneur approprié, tel qu'un vérin, et, le cas échéant tout système de transmission approprié.

Le système d'assistance 1 comprend également deux organes d'étirage 30 comprenant chacun un bras de liaison 31 s'étendant selon une première direction transversale T1, perpendiculaire à l'axe longitudinal L, depuis l'un des chariots 21 du dispositif d'entraînement 15, au travers de l'une des fentes 11 du support 5, et un corps 32 s'étendant depuis le bras de liaison 31 selon une deuxième direction transversale T2, perpendiculaire à l'axe longitudinal L et à la première direction transversale T1.

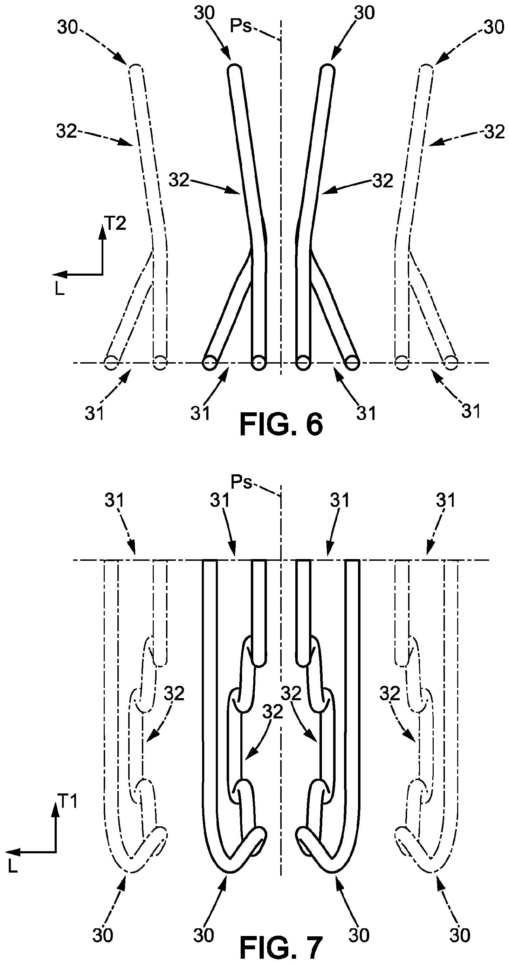

Dans le mode de réalisation représenté, les organes d'étirage 30 sont symétriques l'un par rapport à l'autre selon un plan de symétrie transversal Ps, visible sur les figures 6 et 7.

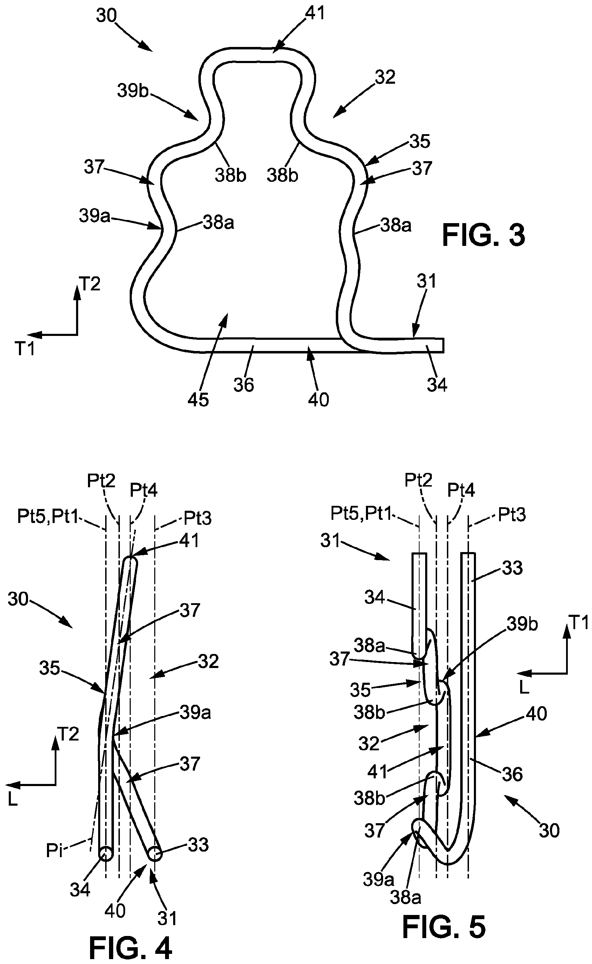

Sur les figures 3 à 5, chacun des organes d'étirage 30 est formé d'un seul tenant par une seule et même tige réalisée en un matériau lui conférant une rigidité d'ensemble une fois mise en forme de manière appropriée comme il sera décrit plus en détails dans ce qui suit. Le matériau peut néanmoins être choisi pour autoriser une déformation élastique du corps 32 par rapport au bras de liaison 31 de telle manière que chacun des organes d'étirage 30 puisse présenter une certaine souplesse selon l'axe longitudinal L. La tige peut, par ailleurs, présenter une surface extérieure adaptée pour faciliter une mise en place du bas de contention 4 sur l'organe d'étirage 30 et/ou assurer un maintien du bas de contention 4 sur l'organe d'étirage 30 comme il apparaîtra de la suite de la description. Par exemple, la tige peut présenter une section ronde, être réalisée en métal, tel que de l'acier ressort, recouvert d'un revêtement, notamment en matière plastique. La tige peut alors être mise en forme par pliage ou cintrage. En variante, la tige peut être mise en forme par moulage, le cas échéant suivi de tout traitement approprié.

Le bras de liaison 31 de l'organe d'étirage 30 est constitué de deux branches de la tige, l'une extérieure 33 et l'autre intérieure 34, s'étendant dans un même plan horizontal, selon la première direction transversale T1 en étant écartées l'une de l'autre selon l'axe longitudinal L.

Le corps 32 de l'organe d'étirage 30 est constitué par un arceau 35 de la tige s'étendant globalement perpendiculairement au plan horizontal des branches extérieure 33 et intérieure 34, selon la deuxième direction transversale T2, entre les branches extérieure 33 et intérieure 34 de la tige. En variante, le bras de liaison 31 de l'organe d'étirage 30 pourrait être constitué d'une seule branche à laquelle l'arceau 35 serait relié de manière appropriée.

Le corps 32 comprend :
- une branche de base 36 s'étendant selon la première direction transversale T1, dans le prolongement de la branche extérieure 33 pour former une base 40 du corps 32 de l'organe d'étirage 30,
- deux branches verticales 37 s'étendant respectivement depuis la branche de base 36 et la branche intérieure 34, globalement selon la deuxième direction transversale T2, en convergeant l'une vers l'autre jusqu'à se rejoindre à l'opposé de la branche de base 36 pour former une extrémité distale 41 de l'organe d'étirage 30.

Les branches verticales 37 du corps 32 comprennent deux premiers bords de retenue 38a, agencés à proximité de la branche de base 36, en regard et écartés l'un de l'autre selon la première direction transversale T1. Chacun des premiers bords de retenue 38a présente une concavité orientée dans une direction opposée à l'autre premier bord de retenue 38a, vers l'extérieur, de telle manière que les premiers bords de retenue 38a sont rapprochés l'un de l'autre, tout en maintenant un premier écartement entre eux. Les premiers bords de retenue 38a forment ainsi une première partie de retenue 39a agencée entre la base 40 et l'extrémité distale 41 de l'organe d'étirage 30 et adaptée pour recevoir et retenir une partie du bas de contention 4, au moins au niveau d'une ouverture d'enfilage de celui-ci, sur l'organe d'étirage 30.

Les branches verticales 37 du corps 32 comprennent également deux deuxièmes bords de retenue 38b, agencés à distance de la branche de base 36, en regard et écartés l'un de l'autre selon la première direction transversale T1. Chacun des deuxièmes bords de retenue 38b présente une concavité orientée dans une direction opposée à l'autre deuxième bord de retenue 38b, vers l'extérieur, de telle manière que les deuxièmes bords de retenue 38b sont rapprochés l'un de l'autre, tout en maintenant un deuxième écartement entre eux, inférieur au premier écartement. Les deuxièmes bords de retenue 38b forment ainsi une deuxième partie de retenue 39b agencée entre la base 40 et l'extrémité distale 41 de l'organe d'étirage 30 et adaptée pour recevoir et retenir une partie du bas de contention 4, au moins au niveau de l'ouverture d'enfilage de celui-ci, sur l'organe d'étirage 30.

En variante, le corps 32 de chaque organe d'étirage 30 pourrait comprendre une seule partie de retenue ou plus de deux parties de retenue dont l'écartement entre les bords de retenue diminue en s'éloignant de la base 40.

Sur les figures 4 et 5, les première 39a et deuxième 39b parties de retenue sont disposées respectivement dans des premier Pt1 et deuxième Pt2 plans transversaux, c'est-à-dire perpendiculaires à l'axe longitudinal L, écartés vers l'intérieur, c'est à-dire en direction de la branche intérieure 34, par rapport à un troisième plan transversal Pt3 dans lequel s'étend la base 40 du corps 32. Les premier Pt1 et deuxième Pt2 plans transversaux sont, par ailleurs, écartés vers l'intérieur par rapport à un quatrième plan transversal Pt4 dans lequel s'étend l'extrémité distale 41 du corps 32.

En particulier, la branche verticale 37 du corps 32 attenante à la branche de base 36 comporte une première portion entre la branche de base 36 et le premier bord de retenue 38a, laquelle première portion est inclinée depuis le troisième plan transversal Pt3 comprenant la base 40 du corps 32 jusqu'à un cinquième plan transversal Pt5 comprenant la branche intérieure 34 du bras de liaison 31. La branche verticale 37 du corps 32 attenante à la branche intérieure 34 comprend une première portion entre la branche intérieure 34 et le premier bord de retenue 38a, laquelle première portion s'étend dans le cinquième plan transversal Pt5. La première partie de retenue 39a est alors disposée sensiblement dans le cinquième plan transversal Pt5 confondu avec le premier plan transversal Pt1. Les branches verticales 37 du corps 32 sont ensuite coudées vers l'extérieur, c'est-à-dire en direction de la branche extérieure 33, de manière à présenter respectivement des deuxièmes portions situées dans un même plan incliné Pi autour d'un axe parallèle à la première direction transversale T1. Le deuxième plan transversal Pt2 comprenant la deuxième partie de retenue 39b est alors situé entre le troisième plan transversal Pt3 comprenant la base 40 et le premier plan transversal Pt1 comprenant la première partie de retenue 38a et la branche intérieure 34. Le quatrième plan transversal Pt4 comprenant l'extrémité distale 41 du corps 32 est situé entre le troisième plan transversal Pt3 comprenant la base 40 et le deuxième plan transversal Pt2 comprenant la deuxième partie de retenue 39b.

L'organe d'étirage 30 présente ainsi une concavité tournée vers l'extérieur et réalisée, dans le mode de réalisation représenté, par un pli s'étendant selon la première direction transversale T1 au niveau de la première partie de retenue 39a.

Le corps 32 de l'organe d'étirage 30, délimité par l'arceau 35 de la tige, est creux et présente un évidement 45 selon la deuxième direction transversale T2 qui s'étend, dans le mode de réalisation représenté, depuis la base 40 jusqu'à l'extrémité distale 41.

Sur les figures 6 et 7, les organes d'étirage 30 sont montés sur les chariots 21, en regard l'un de l'autre, les branches intérieures 34 des bras de liaison 31 étant placées en regard l'une de l'autre.

La branche intérieure 34 du bras de liaison 31 de chaque organe d'étirage 30 se trouve alors écartée de la branche extérieure 33 selon l'axe longitudinal L. En outre, chacune des parties de retenue 39a, 39b de l'un, premier, des organes d'étirage 30 est décalée selon l'axe longitudinal L en direction de la partie de retenue 39a, 39b correspondante de l'autre, deuxième, organe d'étirage 30 par rapport à la base 40 du premier organe d'étirage 30. De la même manière, chacune des parties de retenue 39a, 39b du premier organe d'étirage 30 est décalée selon l'axe longitudinal L en direction de la partie de retenue 39a, 39b correspondante du deuxième organe d'étirage 30 par rapport à l'extrémité distale 41 du premier organe d'étirage 30. Le premier organe d'étirage 30 présente, par ailleurs, une concavité orientée vers l'extérieur, c'est-à-dire dans une direction opposée au deuxième organe d'étirage 30, au niveau de la première partie de retenue 38a. L'écartement selon l'axe longitudinal L entre les bases 40 des organes d'étirage 30 est donc supérieur à l'écartement selon l'axe longitudinal L entre les parties de retenue 39a, 39b. De façon analogue, l'écartement selon l'axe longitudinal L entre les extrémités distales 41 des organes d'étirage 30 est supérieur à l'écartement selon l'axe longitudinal L entre les parties de retenue 39a, 39b.

L'invention a été décrite en relation avec un mode de réalisation dans lequel chacun des organes d'étirage 30 présente la conformation particulière selon l'invention de sorte à pouvoir former tour à tour le premier organe d'étirage 30 et le deuxième organe d'étirage 30. L'invention n'est, toutefois, pas limitée à un tel mode de réalisation et s'applique également à un mode de réalisation dans lequel seul l'un des organes d'étirage 30 présente une conformation particulière selon l'invention et forme le premier organe d'étirage 30, l'autre organe étirage formant le deuxième organe d'étirage 30. En outre, le ou les organes d'étirage 30 pourraient être réalisés de toute autre manière appropriée. En particulier, seul le corps 32 pourrait être formé par la tige dont des extrémités sont reliées à des éléments de liaison intérieur et extérieur s'étendant parallèlement l'un par rapport à l'autre selon la première direction transversale T1 de telle manière que l'écartement entre les éléments de liaison extérieurs soit supérieur à celui des éléments de liaison intérieurs.

En actionnant le ou les boutons de commande 22, les chariots 21 peuvent être déplacés en translation selon l'axe longitudinal L l'un par rapport à l'autre pour placer les organes d'étirage 30 dans une position relative appropriée choisie parmi une pluralité de positions relatives. En particulier, les organes d'étirage 30 peuvent être placés dans une première position relative, rapprochée et centrée par rapport au support 5, apparaissant en traits pleins sur les figures 6 et 7 et correspondant à la position illustrée sur la figure 1. En variante, dans la position rapprochée, les organes d'étirage 30 peuvent venir en contact l'un avec l'autre au niveau des branches intérieures 34 ou de la première partie de retenue 39a. Ils pourraient également être excentrés par rapport au support 5. Les organes d'étirage 30 peuvent être écartés l'un de l'autre vers une deuxième position relative, apparaissant en traits mixtes sur les figures 6 et 7 et correspondant à la position illustrée sur la figure 2.

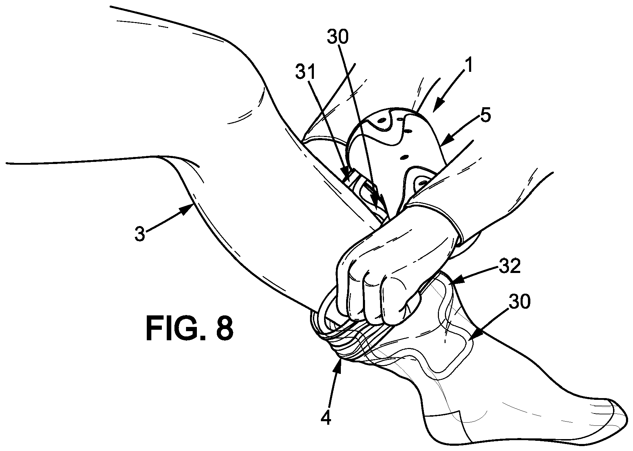

Comme représenté sur la figure 8, après les avoir placés dans la première position relative, rapprochée, les organes d'étirage 30 sont introduits dans l'ouverture d'enfilage du bas de contention 4 par l'extrémité distale 41 jusqu'à ce qu'au moins une partie du bas de contention 4 au voisinage de l'ouverture d'enfilage soit positionnée sur les premières parties de retenue 39a des corps 32 des organes d'étirage 30. Les organes d'étirage 30 peuvent ensuite être écartés l'un de l'autre en actionnant les boutons de commande 22 pour étirer le bas de contention 4 jusqu'à atteindre la position relative permettant de faire passer le pied entre les bases 40 des corps 32 des organes d'étirage 30. L'introduction du pied dans l'espace entre les organes d'étirage 30, dans les évidements 45, termine l'écartement du bas de contention 4, par un enveloppement en douceur de sa partie tarsienne et métatarsienne. L'écartement entre les extrémités distales 41 des corps 32 des organes d'étirage 30 simplifie également le passage du pied entre les organes d'étirage 30. Du fait de la certaine souplesse des organes d'étirage 30, les corps 32, légèrement rapprochés l'un de l'autre sous l'effet d'une contrainte exercée par le bas de contention 4, peuvent être rappelés élastiquement vers un état de repos et s'écarter légèrement l'un de l'autre au fur et à mesure que le bas de contention 4 est enfilé sur le membre inférieur 3 du patient et que la contrainte qu'il exerce diminue.

## Revendications

1. Système d'assistance (1) pour enfiler un dispositif de compression médicale (4) sur un membre (3) d'un patient, le dispositif de compression médicale (4) présentant une ouverture d'enfilage, le système d'assistance (1) comprenant :
- un support (5) s'étendant selon un axe longitudinal (L),
- deux organes d'étirage (30) comprenant chacun un bras de liaison (31) s'étendant depuis le support (5) selon une première direction transversale (T1), et un corps (32) s'étendant depuis le bras de liaison (31) selon une deuxième direction transversale (T2), le corps (32) de chacun des organes d'étirage (30) comportant une base (40) reliée au bras de liaison (31) et au moins une partie de retenue (39a, 39b) écartée de la base (40) et sur laquelle au moins une partie du dispositif de compression médicale (4) au voisinage de l'ouverture d'enfilage est destinée à être positionnée, les organes d'étirage (30) étant montés sur le support (5) en regard l'un de l'autre et déplaçables l'un par rapport à l'autre en translation selon l'axe longitudinal (L),
le système d'assistance (1) étant **caractérisé en ce que** la partie de retenue (39a, 39b) et la base (40) d'au moins l'un, premier, des organes d'étirage (30) s'étendent respectivement dans des plans transversaux (Pt1, Pt2, Pt3) perpendiculaires à l'axe longitudinal (L), le plan transversal (Pt1, Pt2) contenant la partie de retenue (39a, 39b) du premier organe d'étirage (30) étant décalé selon l'axe longitudinal (L) en direction de la partie de retenue (39a, 39b) de l'autre, deuxième, organe d'étirage (30) par rapport au plan transversal (Pt3) contenant la base (40) dudit premier organe d'étirage (40), le corps (32) du premier organe d'étirage (30) présentant un évidement (45) selon la deuxième direction transversale (T2) au moins au niveau de la partie de retenue (39a, 39b),
et **en ce que** le premier organe d'étirage (30) comprend :
- une tige mise en forme pour former le corps (32) et comprenant la base (40) du corps (32),
- un élément de liaison extérieur (33) s'étendant selon la première direction transversale (T1) pour former une partie du bras de liaison (31), la base (40) du corps (32) étant reliée à l'élément de liaison extérieur (33), et
- un élément de liaison intérieur (34) s'étendant selon la première direction transversale (T1) parallèlement à l'élément de liaison extérieur (33), l'élément de liaison intérieur (34) étant écarté de l'élément de liaison extérieur (33) selon l'axe longitudinal (L) en direction du deuxième organe d'étirage (30), l'élément de liaison intérieur (34) formant une autre partie du bras de liaison (31) à laquelle la tige est reliée à l'opposé de la base (40).

2. Système d'assistance (1) selon la revendication 1, dans lequel le corps (32) du premier organe d'étirage (30) présente une concavité orientée dans une direction opposée au deuxième organe d'étirage (30) au niveau de la partie de retenue (39a, 39b) dudit premier organe d'étirage (30).

3. Système d'assistance (1) selon la revendication 2, dans lequel la partie de retenue (39a, 39b) du premier organe d'étirage (30) présente un pli s'étendant selon la première direction transversale (T1).

4. Système d'assistance (1) selon l'une quelconque des revendications 1 à 3, dans lequel le corps (32) de chacun des organes d'étirage (30) présente une extrémité distale (41) opposée à la base (40), la partie de retenue (39a, 39b) du premier organe d'étirage (30) étant décalée selon l'axe longitudinal (L) en direction de la partie de retenue (39a, 39b) du deuxième organe d'étirage (30) par rapport à l'extrémité distale (41) dudit premier organe d'étirage (30).

5. Système d'assistance (1) selon la revendication 4, dans lequel l'extrémité distale (41) du premier organe d'étirage (30) est disposée entre deux plans transversaux (Pt1, Pt3) dans lesquels s'étendent respectivement la base (40) et la partie de retenue (39a, 39b) dudit premier organe d'étirage (30).

6. Système d'assistance (1) selon l'une quelconque des revendications 1 à 5, dans lequel le premier organe d'étirage (30) est formé d'un seul tenant par la tige mise en forme, la tige comportant une branche extérieure (33) formant l'élément de liaison extérieur, et un arceau (35) s'étendant globalement selon la deuxième direction transversale (T2) pour former le corps (32), l'arceau (35) comprenant une branche de base (36) s'étendant dans le prolongement de la branche extérieure (33) pour former la base (40) du corps (32).

7. Système d'assistance (1) selon la revendication 6, dans lequel la tige comporte en outre une branche intérieure (34) formant l'élément de liaison intérieur.

8. Système d'assistance (1) selon la revendication 6 ou 7, dans lequel la tige est réalisée en acier ressort.

9. Système d'assistance (1) selon l'une quelconque des revendications 6 à 8, dans lequel la tige présente une section ronde.

10. Système d'assistance (1) selon l'une quelconque des revendications 1 à 9, dans lequel la partie de retenue (39a, 39b) de chacun des organes d'étirage (30) comporte deux bords de retenue (38a, 38b) en regard écartés l'un de l'autre selon la première direction transversale (T1), chacun des bords de retenue (38a, 38b) présentant une concavité orientée dans une direction opposée à l'autre bord de retenue (38a, 38b).

11. Système d'assistance (1) selon l'une quelconque des revendications 1 à 10, dans lequel le corps (32) de chacun des organes d'étirage (30) comporte une pluralité de parties de retenue (39a, 39b) présentant chacune un écartement entre les bords de retenue (38a, 38b), l'écartement entre les bords de retenue (38a) de l'une des parties de retenue (39a) située à proximité de la base (40) étant supérieur à l'écartement entre les bords de retenue (38b) de l'une des parties de retenue (39b) située à distance de la base (40).

12. Système d'assistance (1) selon l'une quelconque des revendications 1 à 11, comprenant en outre un dispositif d'entraînement (15) logé dans le support (5) et adapté pour déplacer les organes d'étirage (30) l'un par rapport à l'autre, et un organe d'activation (22) placé sur le support (5) et actionnable par un utilisateur pour activer le dispositif d'entraînement (15).

13. Système d'assistance (1) selon la revendication 12, dans lequel le support (5) est adapté pour être porté à la main par l'utilisateur.

## Patentansprüche

1. Hilfssystem (1) zum Anbringen einer medizinischen Kompressionsvorrichtung (4) an einem Glied (3) eines Patienten, wobei die medizinische Kompressionsvorrichtung (4) eine Anbringungsöffnung aufweist, wobei das Hilfssystem (1) Folgendes umfasst:
- einen Träger (5), der sich entlang einer Längsachse (L) erstreckt,
- zwei Streckeinrichtungen (30), die jede einen Verbindungsarm (31), der sich vom Träger (5) in einer ersten Querrichtung (T1) erstreckt, und einen Körper (32), der sich vom Verbindungsarm (31) in einer zweiten Querrichtung (T2) erstreckt, umfassen, wobei der Körper (32) jeder der Streckeinrichtungen (30) eine mit dem Verbindungsarm (31) verbundene Basis (40) und zumindest einen von der Basis (40) beabstandeten Halterungsteil (39a, 39b) umfasst, an dem zumindest ein Teil der medizinischen Kompressionsvorrichtung (4) in der Nähe der Anbringungsöffnung positioniert werden soll, wobei die Streckeinrichtungen (30) einander zugewandt und zueinander entlang der Längsachse (L) verschiebbar am Träger (5) montiert sind,
wobei das Hilfssystem (1) **dadurch gekennzeichnet ist, dass** sich der Halterungsteil (39a, 39b) und die Basis (40) zumindest einer ersten der Streckeinrichtungen (30) jeweils in Querebenen (Pt1, Pt2, Pt3) erstrecken, die senkrecht zur Längsachse (L) sind, wobei die Querebene (Pt1, Pt2), die den Halterungsteil (39a, 39b) der ersten Streckeinrichtung (30) enthält, entlang der Längsachse (L) in Richtung des Halterungsteils (39a, 39b) der anderen zweiten Streckeinrichtung (30) in Bezug auf die Querebene (Pt3), welche die Basis (40) der ersten Streckeinrichtung (40) enthält, versetzt ist, wobei der Körper (32) der ersten Streckeinrichtung (30) eine Ausnehmung (45) entlang der zweiten Querrichtung (T2) zumindest in Höhe des Halterungsteils (39a, 39b) aufweist,
und dadurch, dass die erste Streckeinrichtung (30) Folgendes umfasst:
- einen Formgebungsschaft zum Formen des Körpers (32), umfassend die Basis (40) des Körpers (32),
- ein äußeres Verbindungselement (33), das sich entlang der ersten Querrichtung (T1) erstreckt, um einen Teil des Verbindungsarms (31) auszubilden, wobei die Basis (40) des Körpers (32) mit dem äußeren Verbindungselement (33) verbunden ist, und
- ein inneres Verbindungselement (34), das sich entlang der ersten Querrichtung (T1) parallel zum äußeren Verbindungselement (33) erstreckt, wobei das innere Verbindungselement (34) entlang der Längsachse (L) in Richtung der zweiten Streckeinrichtung (30) vom äußeren Verbindungselement (33) beabstandet ist, wobei das Verbindungselement (34) einen anderen Teil des Verbindungsarms (31) ausbildet, mit dem der Schaft gegenüber der Basis (40) verbunden ist.

2. Hilfssystem (1) nach Anspruch 1, wobei der Körper (32) der ersten Streckeinrichtung (30) eine Konkavität aufweist, die in einer zur zweiten Streckeinrichtung (30) entgegengesetzten Richtung in Höhe des Halterungsteils (39a, 39b) der ersten Streckeinrichtung (30) ausgerichtet ist.

3. Hilfssystem (1) nach Anspruch 2, wobei der Halterungsteil (39a, 39b) der ersten Streckeinrichtung (30) eine Falte aufweist, die sich entlang der ersten Querrichtung (T1) erstreckt.

4. Hilfssystem (1) nach einem der Ansprüche 1 bis 3, wobei der Körper (32) jeder der Streckeinrichtungen (30) ein zur Basis (40) entgegengesetztes distales Ende (41) aufweist, wobei der Halterungsteil (39a, 39b) der ersten Streckeinrichtung (30) entlang der Längsachse (L) in Richtung des Halterungsteils (39a, 39b) der zweiten Streckeinrichtung (30) in Bezug auf das distale Ende (41) der ersten Streckeinrichtung (30) versetzt ist.

5. Hilfssystem (1) nach Anspruch 4, wobei das distale Ende (41) der ersten Streckeinrichtung (30) zwischen zwei Querebenen (Pt1, Pt3) angeordnet ist, in denen sich jeweils die Basis (40) bzw. das Halterungsteil (39a, 39b) der ersten Streckeinrichtung (30) erstrecken.

6. Hilfssystem (1) nach einem der Ansprüche 1 bis 5, wobei die erste Streckeinrichtung (30) integral durch den Formgebungsschaft ausgebildet ist, wobei der Schaft einen äußeren Zweig (33), der das äußere Verbindungselement ausbildet, und einen Bügel (35), der sich im Allgemeinen entlang der zweiten Querrichtung (T2) erstreckt, um den Körper (32) auszubilden, umfasst, wobei der Bügel (35) einen Basiszweig (36) umfasst, der sich in der Verlängerung des äußeren Zweigs (33) erstreckt, um die Basis (40) des Körpers (32) auszubilden.

7. Hilfssystem (1) nach Anspruch 6, wobei der Schaft ferner einen inneren Zweig (34) umfasst, der das innere Verbindungselement ausbildet.

8. Hilfssystem (1) nach Anspruch 6 oder 7, wobei der Schaft aus Federstahl ausgeführt ist.

9. Hilfssystem (1) nach einem der Ansprüche 6 bis 8, wobei der Schaft einen runden Querschnitt aufweist.

10. Hilfssystem (1) nach einem der Ansprüche 1 bis 9, wobei der Halterungsteil (39a, 39b) jeder der Streckeinrichtungen (30) zwei gegenüberliegende Rückhaltekanten (38a, 38b) umfasst, die entlang der ersten Querrichtung (T1) voneinander beabstandet sind, wobei jede der Rückhaltekanten (38a, 38b) eine Konkavität aufweist, die in einer zur anderen Rückhaltekante (38a, 38b) entgegengesetzten Richtung ausgerichtet ist.

11. Hilfssystem (1) nach einem der Ansprüche 1 bis 10, wobei der Körper (32) jeder der Streckeinrichtungen (30) eine Mehrzahl von Rückhalteteilen (39a, 39b) umfasst, die jeder einen Abstand zwischen den Rückhaltekanten (38a, 38b) aufweisen, wobei der Abstand zwischen den Rückhaltekanten (38a) eines der Rückhalteteile (39a), der sich nahe der Basis (40) befindet, größer ist als der Abstand zwischen den Rückhaltekanten (38b) eines der Rückhalteteile (39b), der sich entfernt von der Basis (40) befindet.

12. Hilfssystem (1) nach einem der Ansprüche 1 bis 11, ferner umfassend eine Antriebsvorrichtung (15), die im Träger (5) untergebracht und dazu angepasst ist, die Streckeinrichtungen (30) in Bezug zueinander zu bewegen, und eine Aktivierungseinrichtung (22), die unter dem Träger (5) angeordnet und durch einen Benutzer betätigbar ist, um die Antriebsvorrichtung (15) zu aktivieren.

13. Hilfssystem (1) nach Anspruch 12, wobei der Träger (5) dazu angepasst ist, durch einen Benutzer mit der Hand getragen zu werden.

## Claims

1. Assistance system (1) for fitting a medical compression device (4) onto a limb (3) of a patient, the medical compression device (4) having a fitting opening, with the assistance system (1) comprising:
- a support (5) extending along a longitudinal axis (L),
- two stretching members (30) each comprising a connecting arm (31) extending from the support (5) along a first transversal direction (T1), and a body (32) extending from the connecting arm (31) along a second transversal direction (T2), with the body (32) of each one of the stretching members (30) comprising a base (40) linked to the connecting arm (31) and at least one retaining part (39a, 39b) separated from the base (40) and whereon at least one portion of the medical compression device (4) in the vicinity of the fitting opening is intended to be positioned, with the stretching members (30) being mounted on the support (5) facing one another and which can be displaced with respect to one another in translation along the longitudinal axis (L),
the assistance system (1) being **characterised in that** the retaining part (39a, 39b) and the base (40) of at least one, first, of the stretching members (30) extend respectively in transverse planes (Pt1, Pt2, Pt3) perpendicular to the longitudinal axis (L), with the transversal plane (Pt1, Pt2) containing the retaining part (39a, 39b) of the first stretching member (30) being offset along the longitudinal axis (L) towards the retaining part (39a, 39b) of the other, second, stretching member (30) relative to the transversal plane (Pt3) containing the base (40) of said first stretching member (40), the body (32) of the first stretching member (30) having a recess (45) along the second transversal direction (T2) at least on the retaining part (39a, 39b),
and **in that** the first stretching member (30) comprises:
- a shaped rod for forming the body (32) and comprising the base (40) of the body (32),
- an external connecting element (33) extending along the first transversal direction (T1) for forming a part of the connecting arm (31), the base (40) of the body (32) being linked to the external connecting element (33), and
- an internal connecting element (34) extending along the first transversal direction (T1) parallel to the external connecting element (33), the internal connecting element (34) being separated from the external connecting element (33) along the longitudinal axis (L) in the direction of the second stretching member (30), the internal connecting element (34) forming another part of the connecting arm (31) to which the rod is linked opposite the base (40).

2. Assistance system (1) according to claim 1, wherein the body (32) of the first stretching member (30) has a concavity oriented in a direction opposite the second stretching member (30) on the retaining part (39a, 39b) of said first stretching member (30).

3. Assistance system (1) according to claim 2, wherein the retaining part (39a, 39b) of the first stretching member (30) has a fold extending along the first transversal direction (T1).

4. Assistance system (1) according to any of claims 1 to 3, wherein the body (32) of each one of the stretching members (30) has a distal end (41) opposite the base (40), the retaining part (39a, 39b) of the first stretching member (30) being offset along the longitudinal axis (L) towards the retaining part (39a, 39b) of the second stretching member (30) relative to the distal end (41) of said first stretching member (30).

5. Assistance system (1) according to claim 4, wherein the distal end (41) of the first stretching member (30) is arranged between two transversal planes (Pt1, Pt3) wherein extend respectively the base (40) and the retaining part (39a, 39b) of said first stretching member (30).

6. Assistance system (1) according to any of claims 1 to 5, wherein the first stretching member (30) is formed from a single piece by the shaped rod, the rod comprising an external branch (33) forming the external connecting element, and a cradle (35) extending globally along the second transversal direction (T2) for forming the body (32), the cradle (35) comprising a base branch (36) extending in the extension of the external branch (33) for forming the base (40) of the body (32).

7. Assistance system (1) according to claim 6, wherein the rod further comprises an internal branch (34) forming the internal connecting element.

8. Assistance system (1) according to claim 6 or 7, wherein the rod is made from spring steel.

9. Assistance system (1) according to any of claims 6 to 8, wherein the rod has a round section.

10. Assistance system (1) according to any of claims 1 to 9, wherein the retaining part (39a, 39b) of each one of the stretching members (30) comprises two retaining edges (38a, 38b) facing and separated from one another along the first transversal direction (T1), with each one of the retaining edges (38a, 38b) having a concavity oriented in a direction opposite the other retaining edge (38a, 38b).

11. Assistance system (1) according to any of claims 1 to 10, wherein the body (32) of each one of the stretching members (30) comprises a plurality of retaining parts (39a, 39b) each one having a separation between the retaining edges (38a, 38b), with the separation between the retaining edges (38a) of one of the retaining parts (39a) located in the vicinity of the base (40) being greater than the separation between the retaining edges (38b) of one of the retaining parts (39b) located at a distance from the base (40).

12. Assistance system (1) according to any of claims 1 to 11, further comprising a device for driving (15) housed in the support (5) and adapted to displace the stretching members (30) relative to one another, and an activation member (22) placed on the support (5) and that can be actuated by a user in order to activate the device for driving (15).

13. Assistance system (1) according to claim 12, wherein the support (5) is adapted to be carried by hand by the user.
